# EUROPÄISCHE PATENTSCHRIFT

(11) **EP 3 247 990 B1**
(45) Veröffentlichungstag und Bekanntmachung des Hinweises auf die Patenterteilung: **05.07.2023**
(21) Anmeldenummer: 16700534.7
(22) Anmeldetag: 06.01.2016
(51) Int. Cl.: G01N 21/25, G01N 33/50, G06T 7/254

(54) **VERFAHREN UND VORRICHTUNG ZUR ERMITTLUNG DER WIRKUNG VON WIRKSTOFFEN AN NEMATODEN UND ANDEREN ORGANISMEN IN WÄSSRIGEN TESTS**
METHOD AND DEVICE FOR DETERMINING THE EFFECT OF ACTIVE AGENTS ON NEMATODES AND OTHER ORGANISMS IN AQUEOUS ASSAYS
PROCÉDÉ ET DISPOSITIF DE DÉTERMINATION DE L'ACTION D'AGENTS ACTIFS SUR NÉMATODES ET AUTRES ORGANISMES DANS DES ESSAIS AQUEUX

(30) Priorität: 23.01.2015 EP 15152272
(43) Veröffentlichungstag der Anmeldung: 29.11.2017
(73) Patentinhaber: Bayer Aktiengesellschaft, 51373 Leverkusen (DE)
(72) Erfinder: FOIS, Franco, 40789 Monheim (DE); HARNAU, Michael, 42799 Leichlingen (DE); OCHMANN, Klaus, 51377 Leverkusen (DE)
(74) Vertreter: BIP Patents
(86) Internationale Anmeldenummer: PCT/EP2016/050140
(87) Internationale Veröffentlichungsnummer: WO 2016/116291

(56) Entgegenhaltungen:
- EP-A2- 0 926 483
- EP-A2- 1 686 368
- WO-A1-2006/107864
- CN-A- 1 800 340
- DE-A1- 19 941 167
- GB-A- 2 479 628
- US-A- 5 545 561
- US-A1- 2004 233 545
- US-A1- 2010 184 616
- CHRIS MARCELLINO ET AL: "WormAssay: A Novel Computer Application for Whole-Plate Motion-based Screening of Macroscopic Parasites", PLOS NEGLECTED TROPICAL DISEASES, Bd. 6, Nr. 1, 31. Januar 2012 (2012-01-31) , Seite e1494, XP055202605, DOI: 10.1371/journal.pntd.0001494 in der Anmeldung erwähnt
- BUCKINGHAM STEVEN D. ET AL: "Automated, high-throughput, motility analysis in Caenorhabditis elegans and parasitic nematodes: Applications in the search for new anthelmintics", INTERNATIONAL JOURNAL FOR PARASITOLOGY: DRUGS AND DRUG RESISTANCE, [Online] vol. 4, no. 3, 1 December 2014 (2014-12-01), pages 226-232, XP093015604, ISSN: 2211-3207, DOI: 10.1016/j.ijpddr.2014.10.004 [retrieved on 2023-01-19]

## Beschreibung

Die Erfindung betrifft ein Verfahren zur Ermittlung der Wirkung von Wirkstoffen an Nematoden und anderen Organismen in wässrigen Tests.

Viele Arten von Nematoden (Fadenwürmer) stellen Schädlinge in der Landwirtschaft dar, da sie durch ihr Eindringen in die Wurzelsysteme den Pflanzenstoffwechsel stark beeinträchtigen können. Gegen einen Nematodenbefall sind bereits verschiedene chemische Substanzen, die sogenannten Nematiziden, entwickelt worden. Jedoch besteht ein großer Bedarf, weitere Wirkstoffe zu identifizieren, durch die Nematoden wirkungsvoll bekämpft werden können.

Aus einer Veröffentlichung von Macellino, Gut et al. (Marcellino C, Gut J, Lim KC, Singh R, McKerrow J, et al. (2012) WormAssay: A Novel Computer Application for Whole-Plate Motion-based Screening of Macroscopic Parasites. PLoS Negl Trop Dis 6(1): e1494. doi:10.1371/journal.pntd.0001494) ist eine Vorrichtung zur Ermittlung von der Wirkung von Wirkstoffen an Würmern bekannt, die eine Halterung für eine Zellkulturplatte mit mehreren Kavitäten umfasst, in denen sich die Würmer mit den Wirkstoffen füllen lassen. Die Zellkulturplatte weist dabei eine Unterseite, eine Oberseite sowie Seitenwände auf, die sich zwischen Unterseite und Oberseite der Zellkulturplatte erstrecken. Die Vorrichtung umfasst des Weiteren eine Kamera, die dazu dient, Bilder von der Unterseite der Zellkulturplatte aufzunehmen. Eine Beleuchtungseinrichtung der Vorrichtung weist wenigstens eine Lichtquelle auf, die die Zellkulturplatte ausleuchtet.

GB2479628-A beschreibt ein Verfahren zum Bestimmen der Bewegung eines biologischen Objekts, das das Erfassen einer Zeitserie von Bildern des Objekts unter Verwendung einer Kamera umfasst. Das Objekt kann ein kultivierter Organismus sein, der aus der Gruppe ausgewählt ist, die aus Zebrafischen oder Nematoden besteht.

Es hat sich gezeigt, dass Störeinflüsse wie kondensierte Tropfen an einer Folienabdeckung der Zellkulturplatte die Versuchsergebnisse stark beeinflussen können. Eine schlecht eingestellte Beleuchtungseinrichtung kann dazu führen, dass die Versuchsergebnisse unbrauchbar sind.

Der Erfindung liegt daher die Aufgabe zu Grunde, ein Verfahren zur Ermittlung der Wirkung von Wirkstoffen an Nematoden bereitzustellen, durch die Störungseinflüsse minimiert werden können und somit eine zuverlässige Ermittlung der Wirkung der Wirkstoffe möglich ist.

Gemäß Anspruch 1 ist zwischen einer ersten Lichtquelle und einer ersten Seitenwand der Zellkulturplatte im eingebauten Zustand eine erste optische Einheit angeordnet, die das Licht der ersten Lichtquelle durch die erste Seitenwand in Richtung der Unterseite der Zellkulturplatte lenkt. In einem Ausführungsbeispiel umfasst die optische Einheit eine Linse, die das Licht der ersten Lichtquelle in Richtung der Unterseite der Zellkulturplatte lenkt und/oder bündelt.

Die optische Einheit kann eine Stablinse aufweisen, die sich im Wesentlichen über die gesamte Länge der ersten Seitenwand der Zellkulturplatte erstreckt. Die Stablinse kann sich dabei parallel zur ersten Seitenwand erstrecken. Eine Mittelachse der Stablinse liegt vorzugsweise zwischen der Ebene der Oberseite der Zellkulturplatte und der Ebene der Unterseite der Zellkulturplatte.

Die erste Lichtquelle kann einen Linienlichtleiter aufweisen, der sich vorzugsweise auch über die gesamte Länge der ersten Seitenwand der Zellkulturplatte erstreckt. Somit wird über die gesamte erste Seitenwand Licht in die Zellkulturplatte eingespeist, wobei bedingt durch die Stablinse das Licht in Richtung der Unterseite der Zellkulturplatte gelenkt und/oder gebündelt wird. Der Linienlichtleiter bzw. seine Mittelachse liegt vorzugsweise zwischen der Ebene der Oberseite und der Ebene der Unterseite der von der Halterung getragenen Zellkulturplatte. Die Strahlen des Lichts aus dem Linienlichtleiter verlaufen dabei im Wesentlichen parallel zur Oberseite bzw. Unterseite der Zellkulturplatte und treffen dann auf die Stablinse, durch die das Licht dann in Richtung Unterseite gelenkt wird.

In einem Ausführungsbeispiel verhindert die optische Einheit eine direkte Ausleuchtung einer oberen Kavitätenabdeckung der Zellkulturplatte. Diese obere Kavitätenabdeckung kann beispielsweise in Form einer Folie ausgebildet sein, an der sich Kondenswasser bilden kann. Ein störender Einfluss dieser kondensierten Tropfen auf die Untersuchungsergebnisse kann jedoch durch die gezielte Ausleuchtung der Zellkulturplatte reduziert bzw. vollständig ausgeschlossen werden.

Alternativ oder zusätzlich kann die optische Einheit so ausgebildet sein, dass ein direkter Lichteinfall in ein Objektiv der Kamera verhindert wird. Auch dies hat sich als förderlich für die Qualität und Verlässlichkeit der Untersuchungsergebnisse herausgestellt.

In einem Ausführungsbeispiel der Erfindung beträgt ein Abstand zwischen der ersten Lichtquelle und der Stablinse 2 bis 4 cm. Vorzugsweise ist ein Verstellmechanismus vorgesehen, durch den der Abstand zwischen Lichtquelle und Stablinse frei wählbar innerhalb bestimmter Grenzen eingestellt werden kann. Durch den Verstellmechanismus kann dabei bevorzugt nicht nur der (horizontale) Abstand zwischen Lichtquelle und Stablinse, sondern auch ein Höhenversatz zwischen Lichtquelle und Stablinse eingestellt werden.

In einem Ausführungsbeispiel ist die Halterung aus einem durchsichtigen Material. Vorzugsweise wird ein thermoplastischer Kunststoff wie PMMA (Acrylglas) verwendet.

Die Beleuchtungseinrichtung kann eine zweite Lichtquelle und eine zweite optische Einheit aufweisen, die an einer der ersten Seitenwand gegenüber liegenden zweiten Seitenwand der Zellkulturplatte angeordnet ist. Die zweite optische Einheit kann dabei identisch zur ersten optischen Einheit ausgebildet sein. Auch kann die Anordnung von zweiter Lichtquelle bezogen zur zweiten optischen Einheit der Anordnung entsprechen, wie sie zwischen erster Lichtquelle und erster optischer Einheit gegeben ist.

Aufgrund der Vielzahl von Wirkstoffen, deren Wirkung an Nematoden zu untersuchen ist, besteht ein Bedarf, ein Verfahren mit Hilfe der oben beschriebenen Vorrichtung bereitzustellen, durch die die Wirkstoffe in möglichst kurzer Zeit untersucht werden können. Der Erfindung liegt daher die zusätzliche Aufgabe zu Grunde, ein Verfahren zur Ermittlung der Wirkung von Wirkstoffen an Nematoden bereitzustellen, durch das möglichst viele Wirkstoffe in kurzer Zeit untersucht werden können.

Diese Aufgabe wird mit dem Verfahren gemäß Anspruch 1 gelöst.

Ausführungsbeispiele des erfindungsgemäßen Verfahrens können den von Anspruch 1 abhängigen Ansprüchen entnommen werden.

Das erfindungsgemäße Verfahren zur Ermittlung der Wirkung von Wirkstoffen an Nematoden und anderen Organismen in wässrigen Tests sieht zunächst die Befüllung wenigstens einer Kavität einer Zellkulturplatte mit Nematoden und einem Wirkstoff vor. Die Zellkulturplatte wird dann in eine Vorrichtung gemäß den obigen Ausführungen gesetzt. Es sei an dieser Stelle darauf hingewiesen, dass alternativ die Zellkulturplatte in einer Vorrichtung angeordnet sein kann, die sich von den Ausführungen gemäß den Ansprüche 1 bis 8 unterscheidet.

Von der Zellkulturplatte, vorzugsweise von der Unterseite her, werden nun mehrere, zeitlich aufeinanderfolgende, digitale Bilder erstellt. Diese Bilder werden binarisiert, wobei jedes Pixel eines Bildes zu einer ersten Gruppe (beispielsweise "schwarz") oder einer zweiten Gruppe (beispielsweise "weiß") zugeordnet wird. Bei der Binarisierung kann ein Schwellenwert definiert/festgelegt werden, wobei ein Pixel, dessen Intensität kleiner ist als dieser Schwellenwert, zum Hintergrund gezählt wird, während ein Pixel, dessen Intensität größer als dieser Schwellenwert ist, den Nematoden zugeordnet wird. Die Höhe des Schwellenwerts ist dabei abhängig von der Ausleuchtung der Zellkulturplatte und somit abhängig von der eingesetzten Beleuchtungseinrichtung.

Bevor die Bilder binarisiert werden, kommt zweckmäßig ein morphologischer Bildverarbeitungsfilter zum Einsatz. Dabei werden Objekte, deren Größe größer ist als die der untersuchten Organismen, herausgefiltert. Entsprechend ist für diesen Bearbeitungsschritt eine Eingangsgröße nötig, durch die die Größe der untersuchten Organismen vorgegeben wird.

Nach der Binarisierung wird eine erste Messkurve für eine erste Serie von Aufnahmen ermittelt, wobei sich diese erste Messkurve auf die wenigstens eine Kavität und somit auf den Wirkstoff bezieht, der zuvor in diese Kavität gefüllt worden ist. Die erste Serie von Aufnahmen weist dabei ein Basisbild und mehrere Folgebilder auf, wobei jedes Folgebild im Differenzverfahren mit dem Basisbild verglichen wird und jeweils eine Anzahl von Differenzpixeln bestimmt wird. Des Weiteren wird wenigstens eine auf die Kavität bezogenen zweite Messkurve für einen zweite Serie von Aufnahmen ermittelt, wobei für die zweite Serie ein Folgebild der ersten Serie als Basisbild und wenigstens ein weiteres Folgebild der ersten Serie als ein Folgebild der zweiten Serie verwendet wird. Schließlich erfolgt die Ermittlung einer gemittelten Kurve auf Basis der ersten Messkurve und der wenigstens zweiten Messkurve.

Dadurch, dass ein einziges Bild für verschiedene Messkurven verwendet wird, kann die Anzahl der aufzunehmenden Bilder und somit die dafür benötigte Zeit gering gehalten werden. Durch die Erstellung mehrerer Messkurven, die dann in eine gemittelte Kurve eingehen, kann die statistische Varianz oder Streuung soweit reduziert werden, dass auf Basis der gemittelten Kurve reproduzierbare und verlässliche erste Aussagen zur Wirkung des Wirkstoffes getroffen werden können.

In einer Ausführung der Erfindung wird für das Basisbild der zweiten Serie ein erstes Folgebild der ersten Serie verwendet, das dem Basisbild der ersten Serie unmittelbar folgt (d.h., es gibt keine Bilder, die zwischenzeitlich aufgenommen werden). Weiter werden für die zweite Serie, ausgenommen das erste Folgebild der ersten Serie, alle Folgebilder der ersten Serie als Folgebilder der zweiten Serie verwendet. Die zweite Serie ist dann lediglich durch ein zusätzliches Folgebild zu komplettieren. Besteht beispielsweise die erste Serie aus einem Basisbild und 9 Folgebilder, so werden die 9 Folgebilder der ersten Serie für die zweite Serie verwendet, wobei das erste Folgebild der ersten Serie als Basisbild der zweiten Serie verwendet wird und die übrigen 8 Folgebilder der ersten Serie alle als Folgebilder der zweiten Serie verwendet werden. Damit die zweite Serie ebenfalls 9 Folgebilder aufweist, muss diese durch ein zusätzliches Bild ergänzt werden. Zur Erstellung der ersten Messkurve und der zweiten Messekurve mit jeweils 10 Punkten (einschließlich Nullpunkt), die die Anzahl von Differenzpixeln zu unterschiedlichen Zeitpunkten darstellen, reichen somit insgesamt 11 zeitlich aufeinanderfolgende Bilder aus.

Ein Aufnahmezeitraum zwischen dem Basisbild und dem letzten Folgebild einer Serie kann so festgelegt sein, dass in diesem Aufnahmezeitraum ein asymptotischer Grenzwert für die Anzahl der Differenzpixel für eine Kavität erreicht wird, in der sich unbehandelte Nematoden befinden. Am Beispiel einer Kavität, in der - vereinfachend angenommen - lediglich nur ein Fadenwurm eingefüllt worden ist, soll der Hintergrund des asymptotischen Grenzwerts erläutert werden:
Können beispielsweise diesem einzelnen Fadenwurm 50 weiße Pixel zugeordnet werden, so ist die maximale Anzahl von Differenzpixeln, die sich aus einem Vergleich eines Basisbilds (zum Zeitpunkt t = 0) zu einem danach geschalteten Folgebild ergeben, 100. Die Zahl 100 ergibt sich, wenn sich der Fadenwurm vollständig aus seiner Ursprungslage (Zeitpunkt t = 0) herausbewegt hat. Zum einen sind die ursprünglichen 50 weißen Pixel des Fadenwurms nun schwarz. Zum anderen sind 50 andere Pixel, die zuvor schwarz waren, nun durch die neue Lage des Fadenwurms weiß. Bewegt sich nun dieser Fadenwurm weiter, so bleibt die Anzahl der Differenzpixel aber konstant. Der Aufnahmezeitraum entspricht somit in diesem vereinfachenden Modell der Zeit, die ein unbehandelter Fadenwurm benötigt, sich vollständig aus seiner Ursprungslage (definiert durch das Basisbild) zu bewegen. Vorzugsweise werden in der Regel 50 bis 100 Nematoden in eine Kavität gefüllt, so dass der asymptotische Wert nicht zwangsläufig dem Zweifachen der als "weiß" eingestuften Pixel entsprechen muss. Jedoch zeigt sich, dass die Anzahl der Differenzpixel gegen einen asymptotischen Grenzwert läuft.

Zwischen dem Basisbild der ersten Serie und einem letzten Folgebild einer letzten Serie kann ein Gesamtzeitraum festgelegt werden, der in etwa dem Zweifachen eines Aufnahmezeitraums entsprechen kann (beispielsweise in einem Bereich zwischen 1,5 und 2,5).

Der zeitliche Abstand zwischen zwei aufeinanderfolgende Bilder kann jeweils konstant sein und 1 bis 5 Sekunden betragen. Eine Serie von Bildern kann 8 bis 12 Bilder umfassen. Die Ermittlung der gemittelten Kurve kann auf Basis von 8 bis 12 Messkurven erfolgen.

Wird beispielsweise für eine Kavität mit unbehandelten Nematoden ein Aufnahmezeitraum von 30 Sekunden ermittelt, in dem der asymptotische Grenzwert erreicht wird, so kann der Gesamtzeitraum 60 Sekunden betragen. Bei einem Abstand von drei Sekunden zwischen zwei zeitlich aufeinander folgenden Bildern ergibt sich somit für einen Aufnahmezeitraum eine Anzahl von 11 Bildern pro Serie (ein Basisbild und 10 Folgebilder, wobei das Basisbild zum Zeitpunkt t = 0 aufgenommen wird). Bei einem Gesamtzeitraum von 60 Sekunden, in dem zum Zeitpunkt t = 0 das erste Bild und zum Zeitpunkt t = 60 Sekunden das letzte Bild aufgenommen wird, ergeben sich somit insgesamt 21 Bilder, aus denen sich dann 11 Messkurven mit jeweils 11 Messpunkten (inklusive Nullpunkt) ergeben.

Anhand der in den Figuren dargestellten Ausführungsbeispiele soll die Erfindung näher erläutert werden. Es zeigen:
- Figur 1: schematisch im Querschnitt ein Ausführungsbeispiel für die Vorrichtung;
- Figur 2: schematisch die Anordnung einer Zellkulturplatte und einer Beleuchtungseinrichtung der Vorrichtung gemäß Figur 1;
- Figur 3: ein Ablaufdiagramm eines Ausführungsbeispiels für das erfindungsgemäße Verfahren; und
- Figur 4: eine Messkurve für eine Kavität mit unbehandelten Nematoden und eine Messkurve für mit einem Wirkstoff behandelten Nematoden.

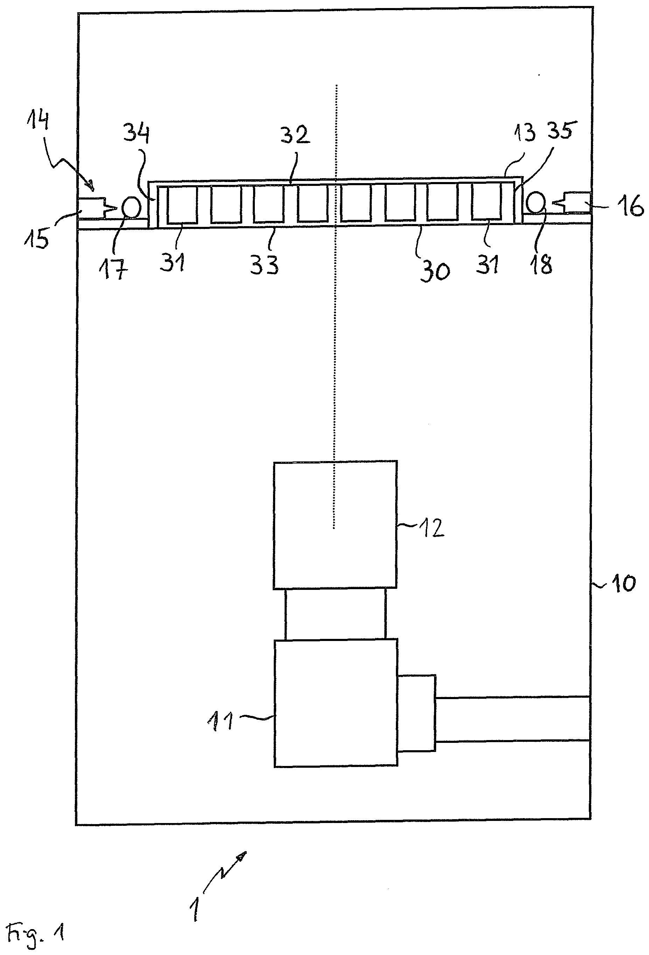

Figur 1 zeigt schematisch im Querschnitt eine Vorrichtung zur Ermittlung der Wirkung von Wirkstoffen an Nematoden. Die Vorrichtung 1 weist ein Gehäuse 10 auf, in dem eine Kamera 11 mit einem Objektiv 12 angeordnet ist. Des Weiteren ist in dem Gehäuse 10 eine Halterung 13 für eine Zellkulturplatte 30 vorgesehen, die mehrere Kavitäten 31 umfasst. Die Zellkulturplatte 30 weist eine Oberseite 32 und eine Unterseite 33 auf. Zwischen der Oberseite 32 und der Unterseite 33 der rechteckigen Zellkulturplatte 30 sind vier Seitenwände angeordnet, von denen in der Darstellung der Figur 1 eine erste Seitenwand 34 und eine gegenüberliegende zweite Seitenwand 35 zu erkennen sind.

Eine Beleuchtungseinrichtung 14, die eine erste Lichtquelle 15 und eine zweite Lichtquelle 16 aufweist, ist ebenfalls im Gehäuse 10 angeordnet. Zwischen der ersten Lichtquelle 15 und der ersten Seitenwand 34 ist als Teil einer ersten optischen Einheit eine Stablinse 17 angeordnet, die sich wie die erste Lichtquelle 15 über die gesamte Länge der ersten Seitenwand 34 erstreckt. Auch zwischen der zweiten Lichtquelle 16 und der zweiten Seitenwand 35 ist eine Stablinse 18 angeordnet.

Durch die Vorrichtung 10 ist es möglich, mit der Kamera 11 mehrere, zeitlich aufeinanderfolgende digitale Bilder von der Zellkulturplatte 30 zu erstellen, wobei die Bilder von der Unterseite 33 der Zellkulturplatte 30 aufgenommen werden. Entsprechend ist die Kamera 11 mit ihrem Objektiv 12 unterhalb der Halterung 13 für die Zellkulturplatte 30 angeordnet. Die Vorrichtung 10 weist hier nicht weiter dargestellte Mittel auf, um die durch die Kamera 11 aufgenommenen Bilder abzuspeichern und zu verarbeiten. Alternativ kann die Vorrichtung 10 mit entsprechenden (Computer-)Mitteln verbunden sein.

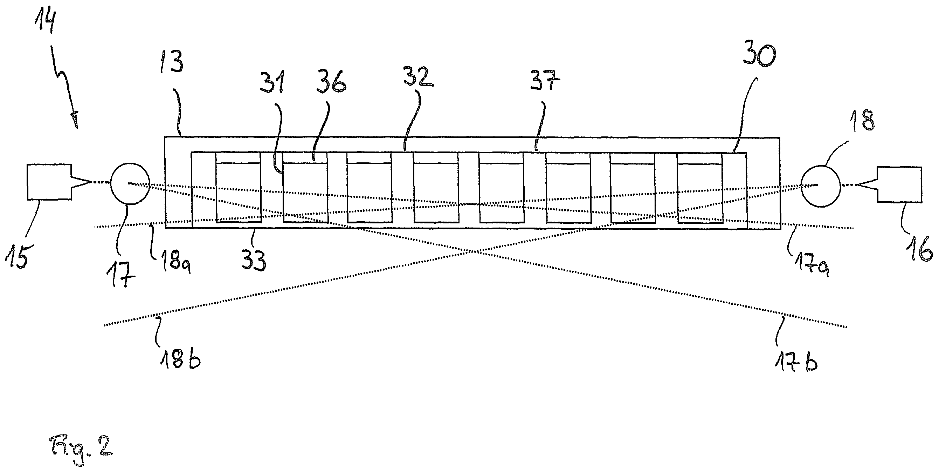

Figur 2 zeigt im vergrößerten Maßstab die Anordnung der Zellkulturplatte 30 und der Beleuchtungseinrichtung 14 mit der ersten Lichtquelle 15 und der zweiten Lichtquelle 16. Die einzelnen Kavitäten 31 der Zellkulturplatte 30 sind mit einer wässrigen Lösung 36 gefüllt, in der sich 50 bis 100 Nematoden und ein zu untersuchender Wirkstoff befinden. In der Figur 2 sind acht in einer Reihe angeordnete Kavitäten 31 dargestellt, wobei sich bei zwölf nebeneinander angeordneten Reihen insgesamt 96 einzelne Kavitäten 31 ergeben würden. Andere Raster für die Zellkulturplatte 30 sind möglich, beispielsweise ein Raster von 4x6 oder 6x8.

In den unterschiedlichen Kavitäten 31 können unterschiedliche Wirkstoffe eingefüllt sein. Auch kann es Kavitäten geben, in denen sich in der wässrigen Lösung nur Nematoden ohne Wirkstoff befinden.

Die Stablinsen 17, 18 bewirken, dass das Licht der Lichtquellen 15, 16 in Richtung der Unterseite 33 gelenkt wird. Die Stablinsen 17, 18 verhindern dabei, dass das Licht der Lichtquellen 15, 16 unmittelbar auf die Oberseite 32 der Zellkulturplatte 30 gelangen kann, wobei an der Oberseite 32 eine Folie 37 vorgesehen ist, die die einzelnen Kavitäten 31 von oben abdeckt. Auch sind die Stablinsen 17, 18 bzw. die Anordnung der Stablinsen 17, 18 so ausgelegt, dass kein Licht unmittelbar in das Objektiv 12 der Kamera 11 fällt. Mit 17a und 17b sind Lichtstrahlen bezeichnet, die aus der Stablinse 17 heraustreten. Entsprechende Austrittsstrahlen der Stablinse 18 sind durch 18a, 18b gekennzeichnet.

Aus der Figur 2 geht hervor, dass die Stablinsen 17, 18, zumindest deren Mittelachsen, die sich senkrecht zur Zeichenebene erstrecken, zwischen der Oberseite 32 und der Unterseite 33 der Zellkulturplatte 30 angeordnet sind, wenn sich die Zellkulturplatte 30 in der für sie vorgesehenen Halterung 13 der Vorrichtung 10 befindet.

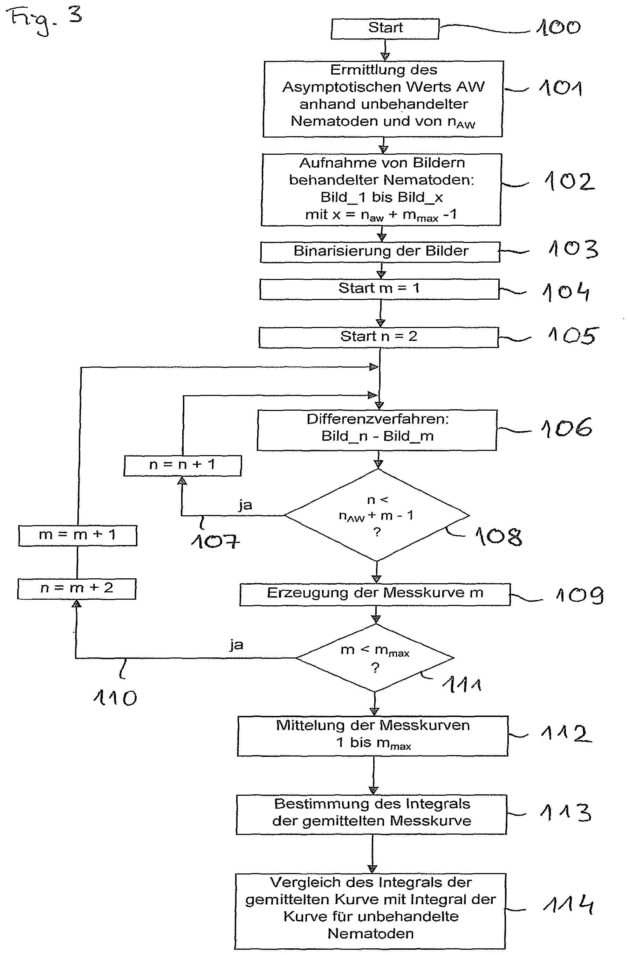

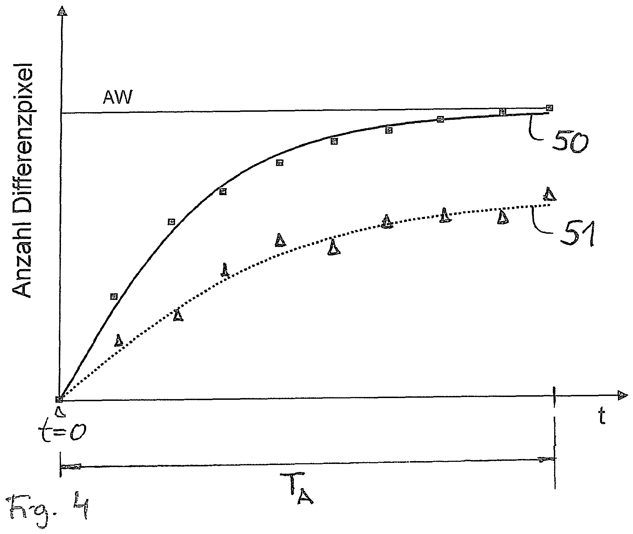

Figur 3 zeigt ein Ablaufdiagramm eines Ausführungsbeispiels für das erfindungsgemäße Verfahren. Das Ablaufdiagramm beginnt mit einem Startblock 100. In Block 101 wird ein asymptotischer Wert AW anhand unbehandelter Nematoden bestimmt. Der asymptotische Wert AW ist dabei die Anzahl von Differenzpixeln, die sich maximal ergeben können, wenn ein Folgebild mit einem Basisbild verglichen wird. Figur 4 zeigt einen solchen asymptotischen Wert AW für eine Messkurve 50 von unbehandelten Nematoden. Die einzelnen Messpunkte geben dabei die Anzahl der Differenzpixel wieder, die sich im Vergleich der zu unterschiedlichen Zeitpunkten aufgenommenen Folgebilder zu einem Basisbild, das zum Zeitpunkt t = 0 aufgenommen worden ist, ergeben. Mit dem asymptotischen Wert AW ist dabei ein Aufnahmezeitraum T_{A} verknüpft, innerhalb dessen die Messkurve 50 den asymptotischen Grenzwert AW zumindest näherungsweise erreicht. Im vorliegenden Beispiel soll der Aufnahmezeitraum 27 Sekunden betragen, wobei der Abstand zwischen zwei benachbarten Folgebildern bzw. der Abstand zwischen dem ersten Folgebild und dem Basisbild (t = 0) drei Sekunden betragen soll. Somit ist eine Anzahl n_{AW} von Bildern pro Messkurve einschließlich des Basisbilds (t = 0) gleich 10.

Figur 4 zeigt auch exemplarisch eine (gemittelte) Messkurve 51 für behandelte Nematoden. Zu erkennen ist, dass die Messkurve 51 unterhalb der Messkurve 50 für die unbehandelten Nematoden verläuft, da sich die behandelten Nematoden langsamer oder einige von ihnen nicht mehr bewegen. Je kleiner die Fläche unter der Messkurve 51 bezogen auf die Fläche unterhalb der Messkurve für unbehandelte Nematoden ist, desto stärker ist die Wirkung des entsprechenden Wirkstoffes. Auf die gemittelte Messkurve 51 wird aber noch später näher eingegangen.

Nach der Ermittlung des asymptotischen Werts AW anhand unbehandelte Nematoden und nach der Festlegung der Anzahl der Bilder n_{AW} pro Messkurve bzw. pro Serie (Block 101) erfolgt nun gemäß Figur 3 in Block 102 die Aufnahme einer bestimmten Anzahl von Bildern Bild_1 bis Bild_x. An dieser Stelle sei darauf hingewiesen, dass die Kamera 11 zu einem Zeitpunkt jeweils ein Gesamtbild der Zellkulturplatte 30 mit allen Kavitäten erstellt, wobei dann aus diesem Gesamtbild für jede einzelne Kavität kavitätsbezogen die Bilder Bild_1 bis Bild_x herausgeschnitten bzw. erstellt werden. Diese Bilder werden dann in Block 103 binarisiert. Bei der Binarisierung werden die einzelnen Pixel entweder den Nematoden (weißes Pixel) oder dem Hintergrund (schwarzes Pixel) zugeordnet. Der Binarisierung in Block 103 ist eine Festlegung eines Schwellenwerts für die Intensität des Pixels vorzuschalten, durch den die Pixel in "weiß" und "schwarz" aufgeteilt werden können.

Für eine erste Messkurve m = 1 (siehe Block 104) werden nun die Bilder Bild_2 bis Bild_n_{AW} verwendet, indem durch ein Differenzverfahren die Anzahl der Differenzpixeln zwischen zunächst einem ersten Folgebild Bild_2 und dem Basisbild Bild_1 ermittelt werden (siehe Block 105 und Block 106). Block 106 wird dabei im Rahmen einer Schleife mehrmals durchlaufen, so dass die Anzahl von Differenzpixeln für mehrere Folgebilder ermittelt wird (Bild_2 - Bild_1; Bild_3 - Bild_1; Bild_4 - Bild_1; ...; Bild_n_{AW} - Bild_1). Die Schleife 107 wird verlassen, wenn die Anzahl der Bilder für die erste Messkurve den Wert n = n_{AW} + m -1 erreicht hat und die innerhalb der Schleife 107 vorgesehene Abfrage 108 nicht mit "ja" beantwortet werden kann. In diesem Fall liegen alle Werte vor, um die erste Messkurve m = 1 zu erstellen (vergl. Block 109).

Nach Erstellung der ersten Messkurve m=1 werden nun mittels der Schleife 110 weitere Messkurven erstellt (m = 2, m = 3, ..., m = mₘₐₓ). Eine zweite Messkurve basiert dabei auf den Differenzbildern Bild_3 - Bild_2; Bild_4 - Bild_2; ...; Bild_n_{AW}+1 - Bild_2. Somit wird beispielsweise das Bild_3 sowohl für die Erstellung der ersten Messkurve m = 1 als auch für die zweite Messkurve m = 2 genutzt.

Wenn eine Abfrage 111 innerhalb der Schleife 110 nicht mit "ja" beantwortet werden kann, wird die Schleife 110 verlassen. Es liegen nun alle Messkurven 1, 2, ..., mₘₐₓ vor, sodass eine Mittelung dieser Messreihen in Block 112 erfolgen kann. Es hat sich herausgestellt, dass durch diese Mittelung der einzelnen Messreihen, die zum überwiegenden Teil auf gleiche Bilder zurückgreifen, die statistische Streuung erheblich reduziert werden kann. Somit können mit insgesamt m+n_{AW}-1 Bildern m Messreihen mit jeweils n_{AW} Messpunkten (einschließlich des Nullpunktes) erzeugt werden.

In Block 113 erfolgt die Berechnung des Integrals der gemittelten Kurve, wobei dieses Integral dann mit der Fläche unter der Messkurve 50 für unbehandelte Nematoden verglichen werden kann (siehe Block 114).

Wenn angenommen wird, dass die bereits oben erwähnte Messreihe 51 in Figur 4 der gemittelten Messreihe gemäß Block 112 entspricht und die Fläche unterhalb der Messkurve 50 I₅₀ = 100 Flächeneinheiten und die Fläche unterhalb der (gemittelten) Messkurve I₅₁ = 65 Flächeneinheiten umfassen, so kann über die Wirksamkeit des Wirkstoffes eine Aussage gemäß der folgenden Formel getroffen werden: (I_{50(unbehandelt)} - I_{51(behandelt)}/I_{50(unbehandelt)} ·100%. Bei dem hier zu Grunde gelegten Zahlenbeispiel würde sich dann ein Wert von 35 % ergeben.

Somit können beispielsweise bei einer Zellkulturplatte mit 96 Kavitäten nahezu 100 Wirkstoffe gleichzeitig untersucht werden. Für die Aufnahme der Bilder, die notwendig sind, eine ausreichende Anzahl von Messreihen mit ausreichend vielen Messpunkten zu erzeugen, um statistisch belastbare Ergebnisse zu erzielen, reichen mitunter 60 Sekunden aus. Die Erfindung ermöglicht somit eine schnelle und effiziente Untersuchung der Wirksamkeit von Wirkstoffen an Nematoden oder ähnlichen Organismen.

### Bezugszeichenliste

- 1: Vorrichtung

- 10: Gehäuse
- 11: Kamera
- 12: Objektiv
- 13: Halterung
- 14: Beleuchtungseinrichtung
- 15: Erste Lichtquelle
- 16: Zweite Lichtquelle
- 17: Stablinse (17a, 17b Austrittsstrahlen)
- 18: Stablinse (18a, 18b Austrittsstrahlen)

- 30: Zellkulturplatte
- 31: Kavität
- 32: Oberseite
- 33: Unterseite
- 34: Erste Seitenwand
- 35: Zweite Seitenwand
- 36: Lösung
- 37: Kavitätsabdeckung

- 100: StartBlock
- 101: Block
- 102: Block
- 103: Block
- 104: Block
- 105: Block
- 106: Block
- 107: Schleife
- 108: Abfrage
- 109: Block
- 110: Schleife
- 111: Abfrage
- 112: Block
- 113: Block
- 114: Block

## Patentansprüche

1. Verfahren zur Ermittlung der Wirkung von Wirkstoffen an Nematoden und anderen Organismen in wässrigen Tests, mit folgenden Schritten:
a) Befüllung wenigstens einer Kavität (31) einer Zellkulturplatte (30) mit Nematoden und einem Wirkstoff;
b) Anordnung der Zellkulturplatte (30) in einer Vorrichtung (1) umfassend:
- eine Halterung (13) für die Zellkulturplatte (30), wobei die Zellkulturplatte (30) eine Unterseite (33), eine Oberseite (32) sowie Seitenwände aufweist, die sich zwischen Unterseite (33) und Oberseite (32) erstrecken und die Kavitäten der Zellkulturplatte durch eine Kavitätenabdeckung verschlossen sind,
- eine Kamera (11), die dazu dient, Bilder von der Zellkulturplatte (30) aufzunehmen,
- eine Beleuchtungseinrichtung (14) mit wenigstens einer ersten Lichtquelle (15), die die Zellkulturplatte (30) ausleuchtet; wobei zwischen der ersten Lichtquelle (15) und einer ersten Seitenwand (34) der Zellkulturplatte (30) im eingebauten Zustand eine erste optische Einheit angeordnet ist, die das Licht der ersten Lichtquelle (15) durch die erste Seitenwand (34) in Richtung der Unterseite (33) der Zellkulturplatte (30) lenkt;
c) Erstellung von mehreren, zeitlich aufeinander folgenden Bildern der Zellkulturplatte (30);
d) Binarisierung der erstellten Bilder in Abhängigkeit einer Ausleuchtung der Zellkulturplatte (30);
e) Ermittlung einer auf die Kavität bezogenen ersten Messkurve für eine erste Serie von Bildern, wobei die erste Serie ein Basisbild und mehrere Folgebilder aufweist, wobei jedes Folgebild im Differenzverfahren mit dem Basisbild verglichen wird und eine Anzahl von Differenzpixeln bestimmt wird;
f) Ermittlung wenigstens einer auf die Kavität bezogenen zweiten Messkurve für eine zweite Serie von Bildern, wobei für die zweite Serie ein Folgebild der ersten Serie als Basisbild und wenigstens ein weiteres Folgebild der ersten Serie als ein Folgebild der zweiten Serie verwendet wird;
g) Ermittlung einer gemittelten Kurve auf Basis der ersten Messkurve und der wenigstens zweiten Messkurve.

2. Verfahren nach Anspruch 1, **dadurch gekennzeichnet, dass** für das Basisbild der zweiten Serie ein erstes Folgebild der ersten Serie verwendet wird, das dem Basisbild der ersten Serie unmittelbar folgt, dass für die zweite Serie, ausgenommen vom ersten Folgebild der ersten Serie, alle Folgebilder der ersten Serie als Folgebilder der zweiten Serie verwendet werden und dass die zweite Serie durch ein zusätzliches Folgebild ergänzt wird.

3. Verfahren nach einem der Ansprüche 1 oder 2, **dadurch gekennzeichnet, dass** ein Aufnahmezeitraum (T_{A}) zwischen dem Basisbild und dem letzten Folgebild einer Serie so festgelegt wird, dass in diesem Aufnahmezeitraum (T_{A}) ein asymptotischer Grenzwert AW für die Anzahl der Differenzpixel für eine Kavität erreicht wird, in der sich unbehandelte Nematoden befinden.

4. Verfahren nach Anspruch 3, **dadurch gekennzeichnet, dass** zwischen dem Basisbild der ersten Serie und einem letzten Folgebild einer letzten Serie ein Gesamtzeitraum festgelegt wird, der in etwa dem Zweifachen eines Aufnahmezeitraums (T_{A}) entspricht.

5. Verfahren nach einem der Ansprüche 1 bis 4, **dadurch gekennzeichnet, dass** der zeitliche Abstand zwischen zwei auf einander folgenden Bildern konstant ist und 1 bis 5 Sekunden beträgt.

6. Verfahren nach einem der Ansprüche 1 bis 5, **dadurch gekennzeichnet, dass** eine Serie von Bildern 8 bis 12 Bilder umfasst.

7. Verfahren nach einem der Ansprüche 2 bis 6, **dadurch gekennzeichnet, dass** die Ermittlung der gemittelten Kurve auf Basis von 8 bis 12 Messkurven erfolgt.

8. Verfahren nach einem der Ansprüche 1 bis 7, wobei die optische Einheit eine Stablinse (17) aufweist, die sich im Wesentlichen über die gesamte Länge der ersten Seitenwand (34) der Zellkulturplatte (30) erstreckt.

9. Verfahren nach einem der Ansprüche 1 bis 8, wobei die erste Lichtquelle (15) einen Linienlichtleiter aufweist.

10. Verfahren nach einem der Ansprüche 1 bis 9, wobei die Beleuchtungseinrichtung (13) eine zweite Lichtquelle (16) und eine zweite optische Einheit aufweist, die an einer der ersten Seitenwand gegenüber liegenden zweiten Seitenwand (35) der Zellkulturplatte (30) angeordnet ist.

## Claims

1. Method for determining the action of active ingredients on nematodes and other organisms in aqueous tests, comprising the following steps:
a) filling at least one well (31) of a cell culture plate (30) with nematodes and an active ingredient;
b) arranging the cell culture plate (30) in a device (1) comprising:
- a holder (13) for the cell culture plate (30), said cell culture plate (30) having a bottom side (33), a top side (32) and also side walls extending between bottom side (33) and top side (32), and the wells of the cell culture plate being closed by a well-cover,
- a camera (11) which is used to record images of the cell culture plate (30),
- a lighting mechanism (14) having at least a first light source (15) which illuminates the cell culture plate (30); there being arranged between the first light source (15) and a first side wall (34) of the cell culture plate (30) in the installed state a first optical unit which directs the light of the first light source (15) through the first side wall (34) in the direction of the bottom side (33) of the cell culture plate (30);
c) creating multiple images of the cell culture plate (30) that follow one another chronologically;
d) binarizing the created images depending on an illumination of the cell culture plate (30);
e) determining a first measurement curve based on the well for a first series of images, said first series having a base image and multiple follow-up images, with each follow-up image being compared to the base image in a difference method and a number of difference pixels being determined;
f) determining at least a second measurement curve based on the well for a second series of images, and, for the second series, using a follow-up image of the first series as base image and at least one further follow-up image of the first series as a follow-up image of the second series;
g) determining an averaged curve on the basis of the first measurement curve and of the at least second measurement curve.

2. Method according to Claim 1, **characterized in that** what is used for the base image of the second series is a first follow-up image of the first series, which first follow-up image immediately follows the base image of the first series, **in that** what are used for the second series are all follow-up images of the first series as follow-up images of the second series, except for the first follow-up image of the first series, and **in that** the second series is completed by an additional follow-up image.

3. Method according to either of Claims 1 and 2, **characterized in that** a recording period (T_{A}) between the base image and the last follow-up image of a series is established such that, within said recording period (T_{A}), an asymptotic limit AW is reached for the number of difference pixels for a well in which untreated nematodes are situated.

4. Method according to Claim 3, **characterized in that** a total period is established between the base image of the first series and a last follow-up image of a last series, which total period approximately corresponds to twice that of a recording period (T_{A}).

5. Method according to any of Claims 1 to 4, **characterized in that** the time interval between two successive images is constant and is from 1 to 5 seconds.

6. Method according to any of Claims 1 to 5, **characterized in that** one series of images comprises from 8 to 12 images.

7. Method according to any of Claims 2 to 6, **characterized in that** the averaged curve is determined on the basis of from 8 to 12 measurement curves.

8. Method according to any of Claims 1 to 7, wherein the optical unit has a rod lens (17) which substantially spans the entire length of the first side wall (34) of the cell culture plate (30).

9. Method according to any of Claims 1 to 8, wherein the first light source (15) has a line light guide.

10. Method according to any of Claims 1 to 9, wherein the lighting mechanism (13) has a second light source (16) and a second optical unit, which is arranged on a second side wall (35) of the cell culture plate (30), said second side wall (35) being opposite to the first side wall.

## Revendications

1. Procédé de détermination de l'action de matières actives sur des nématodes et d'autres organismes dans des essais aqueux, comportant les étapes suivantes :
a) remplissage d'au moins un puits (31) d'une plaque de culture cellulaire (30) avec des nématodes et une matière active ;
b) mise en place de la plaque de culture cellulaire (30) dans un dispositif (1) comprenant :
- un support (13) pour la plaque de culture cellulaire (30), la plaque de culture cellulaire (30) comportant une face inférieure (33), une face supérieure (32) ainsi que des parois latérales qui s'étendent entre la face inférieure (33) et la face supérieure (32), et les puits de la plaque de culture cellulaire étant obturés par un couvercle de puits,
- un appareil photographique (11), qui sert à prendre des images de la plaque de culture cellulaire (30),
- un dispositif d'éclairage (14) comportant au moins une première source de lumière (15), qui éclaire la plaque de culture cellulaire (30) ; une première unité optique étant disposée entre la première source de lumière (15) et une première paroi latérale (34) de la plaque de culture cellulaire (30) à l'état monté, unité qui dévie dans la direction de la face inférieure (33) de la plaque de culture cellulaire (30) la première source de lumière (15) à travers la première paroi latérale (34) ;
c) réalisation de plusieurs images successives dans le temps de la plaque de culture cellulaire (30) ;
d) binarisation des images réalisées en fonction d'un éclairage de la plaque de culture cellulaire (30) ;
e) détermination d'une première courbe de mesure se rapportant au puits pour une première série d'images, la première série présentant une image de base et plusieurs images subséquentes, chaque image subséquente étant comparée à l'image de base par une technique de différenciation, et déterminant un certain nombre de pixels de différence ;
f) détermination d'au moins une deuxième courbe de mesure se rapportant au puits pour une deuxième série d'images, une image subséquente de la première série étant utilisée comme image de base pour la deuxième série et au moins une autre image subséquente de la première série étant utilisée comme image subséquente de la deuxième série ;
g) détermination d'une courbe moyennée à base de la première courbe de mesure et de l'au moins une deuxième courbe de mesure.

2. Procédé selon la revendication 1, **caractérisé en ce que**, pour l'image de base de la deuxième série, on utilise une première image subséquente de la première série, qui suit immédiatement l'image de base de la première série ; **en ce que**, pour la deuxième série, à l'exception de la première image subséquente de la première série, toutes les images subséquentes de la première série sont utilisées comme images subséquentes de la deuxième série ; et **en ce que** la deuxième série est complétée par une autre image subséquente.

3. Procédé selon l'une des revendications 1 ou 2, **caractérisé en ce qu'**on définit une période de prise (T_{A}) entre l'image de base et la dernière image subséquente d'une série, de façon à atteindre dans cette période de prise (T_{A}) une limite asymptomatique AW pour le nombre des pixels de différence pour un puits dans lequel se trouvent des nématodes non traités.

4. Procédé selon la revendication 3, **caractérisé en ce qu'**on définit entre l'image de base de la première série et une dernière image subséquente d'une dernière série une période totale qui correspond approximativement au double de la période de prise (T_{A}).

5. Procédé selon l'une des revendications 1 à 4, **caractérisé en ce que** l'écart temporel entre deux images successives est constant et est de 1 à 5 secondes.

6. Procédé selon l'une des revendications 1 à 5, **caractérisé en ce qu'**une série d'images comprend 8 à 12 images.

7. Procédé selon l'une des revendications 2 à 6, **caractérisé en ce que** la détermination de la courbe moyennée s'effectue sur la base de 8 à 12 courbes de mesure.

8. Procédé selon l'une des revendications 1 à 7, dans lequel l'unité optique comprend une lentille cylindrique (17), qui pour l'essentiel s'étend sur toute la longueur de la première paroi latérale (34) de la plaque de culture cellulaire (30).

9. Procédé selon l'une des revendications 1 à 8, dans lequel la première source de lumière (15) est un conduit de lumière linéaire.

10. Procédé selon l'une des revendications 1 à 9, dans lequel un dispositif d'éclairage (13) comprend une deuxième source de lumière (16) et une deuxième unité optique, qui est disposée contre une paroi latérale (35) de la plaque de culture cellulaire (30) opposée à la première paroi latérale.
